# EUROPEAN PATENT APPLICATION

(11) **EP 2 090 664 A1**
(43) Date of publication of application: **19.08.2009**
(21) Application number: 08002878.0
(22) Date of filing: 15.02.2008
(51) Int. Cl.: C12Q 1/68

(54) **Neuroprotective peptides**

(71) Applicant: Universität Konstanz, 78464 Konstanz (DE)
(72) Inventor: Przybylski, Michael, Dr., 78465 Konstanz (DE); Paraschiv, Gabriela, 78467 Konstanz (DE); Juszczyk, Paulina, Dr., 78-200 Bialogard (PL); Szymanska, Aneta, Dr., 80-512 Gdansk (PL); Grzonka, Zbigniew, Dr., 80-279 Gdansk (PL)
(74) Representative: Müller-Boré & Partner Patentanwälte

(57) **Abstract**

The present invention relates to ligands for selectively binding a non-complexed member of the β-amyloid polypeptide (Aβ)/human cystatin C (HCC)-complex, a pharmaceutical composition containing said ligands, methods for detecting non-complexed members of the Aβ/HCC-complex using said ligands as well as to a kit containing said ligands.

## Description

The present invention relates to ligands for selectively binding a non-complexed member of the β-amyloid polypeptide (Aβ)/human cystatin C (HCC)-complex, a pharmaceutical composition containing said ligands, methods for detecting non-complexed members of the Aβ/HCC-complex using said ligands as well as to a kit containing said ligands.

Alzheimer's disease (AD) and other AD-related neurodegenerative disorders have become the most common form of progressive cognitive failure in humans, a development which is accelerating at present due to the remarkable increase in life expectancy during the past decades. AD represents now the most common form of dementia among elderly population (prevalence: 1000/100,000; > 65 years) in the developed world. Key neuropathological features in AD brain are cortical atrophy, neuronal loss, region-specific amyloid deposition, neuritic plaques, and neurofibrillary tangles. The major constituent of the amyloid fibrils deposited in the brains of patients with Alzheimer's disease (AD), Down's syndrome, cerebral amyloid angiopathy (CAA), hereditary cerebral hemorrhage with amyloidosis, Dutch type (HCHWH-D), and in aged individuals without any neurological disorder, is the β-amyloid polypeptide (Aβ). The major constituent of the neuritic plaque, β-amyloid polypeptide (Aβ), arises from a larger precursor protein, the amyloid precursor protein (APP). Aβ is produced by normal cells and can be detected as a circulating peptide in the plasma and cerebrospinal fluid (CSF) of healthy humans. The accumulation of Aβ, a 39-42 amino acid proteolytic product of APP, in neuritic plaques, structures which at autopsy fulfill the neuropathological criteria for a definitive diagnosis of AD, is thought to be causative for disease progression. A major Aβ cleavage product of APP is the Aβ (1-42) polypeptide, but Aβ-peptides shorter at the C-terminus (39 to 41) are also produced by the proteolytic (g-secretase) cleavage in the membrane. The N-terminal part of Aβ (1-42) is localized in the extracellular region of APP, and the major C-terminal part of the Aβ-peptide is contained within the transmembrane domain.

Despite the lack of details on degradation pathways and cellular transport for the formation and deposition of Aβ-derived plaques, recent studies towards the development of immunisation methods of AD based on therapeutically active antibodies produced from Aβ (1-42) have yielded initial success in transgenic mouse models of Alzheimer's disease. Antibodies generated by immunisation with Aβ (1-42) have been found capable of inhibiting the formation of Aβ-plaques by disaggregating Aβ-fibrils, and improve the impairments in the spatial memory of mice. Both immunization and administration of Aβ-antibodies significantly attenuated amyloid plaque deposition, neuritic dystrophy, and astrogliosis. These findings raised the possibility that formation and clearance of Aβ-antigen:antibody complexes may decrease brain Aβ deposition either by antibody generation within the central nervous system, or by peripheral antibody transport across the blood-brain-barrier (BBB). Furthermore, passive immunization appears to reduce brain Aβ burden by altering Aβ equilibrium between the CNS and plasma.

Several amyloidogenic proteins such as cystatin C, apolipoprotein E, clusterin, witronectin, transtitetin, and gelsolin have been found to co-deposit with Aβ-peptides in the brain as elements of amyloid plaques. In particular, the presence of human cystatin C (HCC) in amyloid deposits has been intriguing. The function of HCC in the brain is unclear at present but a wide spectrum of activities have been assumed in conjunction with other protease inhibitors in the central nervous system, such as modulation of neuropeptide activation and degradation, neurite proliferation, and neuronal protection. HCC is overexpressed in the neurons particularly susceptible to AD degenerative processes, including the entorhinal cortex, hippocampus and temporal cortex, and it has been proposed that cystatin C may play a role in AD pathogenesis.

Cystatin C, also called post-gamma or gamma trace, is a 120-amino acid protein (13 kDa) produced by a majority of nuclear cells. The gene for cystatin C is located on chromosome 20. It is a cysteine protease inhibitor found in all mammalian body fluids. The physiological role of cystatin C is likely to regulate extracellular cysteine protease activity during microbial invasion, or release of lysosomal proteinases from dying or diseased cells. The concentration of circulating cystatin C is high, 5 times higher in CSF than in serum. A L68Q mutation variant of cystatin causes hereditary cerebral hemorrhage with amyloidosis-Icelandic type (HCHWA-I). *In vitro* studies suggest a broad spectrum of biological roles such as in bone resorption, inhibition of extracellular cysteine protease activities, modulation of inflammatory responses, stimulation of glomerular mesanglial cells, and fibroblast proliferation. Cystatin C has been also localized in the cytoplasm of brain neurons and in the adrenal medulla, in the thyroid glands, in the anterior pituitary lobe and in A-cells of the pancreas.

Immunohistochemical studies have shown co-localization of HCC with Aβ, predominantly in amyloid-loaded vascular walls and in senile amyloid plaque. Proteins associated with β-amyloid may initiate and/or enhance fibril formation, or alternatively solubilize and/or inhibit Aβ-fibril formation. *In vitro* binding of HCC to Aβ inhibits amyloid fibril formation, and showed that cystatin C directly associates with APP through the extracellular region.

Molecular and genetic studies also support a role of cystatin C in AD, and the HCC gene (CST3) has been proposed as a recessive risk factor for late-onset AD associated with a reduction of cystatin C secretion. Reduced levels of cystatin C could be a consequence of massive recruitment of HCC in the intracellular compartments, mostly in axons, through, an increase of its endocytotic reuptake. The presence of cystatin C in β-amyloid deposits may be the result of HCC binding to the precursor protein prior to Aβ generation, or HCC may bind to Aβ prior to its secretion or following Aβ deposition in brain. It has also been suggested that the interaction between proteases and HCC might cause an imbalance between proteases and their inhibitors in the vessel walls, contributing to degradation of cerebral microvessels. Most recent results showed that co-incubation of HCC with monomeric Aβ (1-42) attenuated the *in vitro* formation of Aβ oligomers, however HCC did not dissolve performed Aβ oligomers. Direct binding of cystatin C to Aβ possibly occurs by formation of a high-affinity 1:1-complex. These observations suggest that cystatin C may be a regulating element in the transformation of monomeric Aβ to larger toxic aggregates *in vivo*.

Major problems and limitations of present immunological methods for diagnosis treatment of neurodegenerative diseases are problems of AD immunotherapy by active vaccination with Aβ (1-42) and/or related pre-aggregated Aβ-immunogens by high toxicity and/or other side effects due to the antibody history produced as well as problems in production and availability of Aβ-autoantibodies for passive immunisation and related stability problems.

Thus, the problem underlying the present invention is to provide a new system/means for a successful diagnosis and therapy of AD or AD-related neurodegenerative disorders.

The solution to the above technical problem is achieved by the embodiments characterized in the claims.

In particular, the present invention relates to a ligand for selectively binding at least one non-complexed member of the β-amyloid polypeptide (Aβ)/human cystatin C (HCC)-complex, comprising at least one amino acid sequence selected from the group consisting of SEQ ID No. 2 and/or SEQ ID No. 3 and/or SEQ ID No. 6 and/or SEQ ID No. 7 and/or biologically active derivatives thereof

The ligand of the present invention does not include polypeptides/proteins being identical to any Aβ-subclones, such as Aβ (1 - 39), Aβ (1 - 40), Aβ (1 - 41), Aβ (1 - 42) or HCC-subclones, such as naturally occurring subclones, which naturally occurs in mammals, particularly human beings. In particular, the ligand according to the present invention does not consist of an amino acid sequence of SEQ ID No. 1, SEQ ID No. 5, or Aβ (25 - 35) or a biologically active derivative thereof.

Further, in a preferred embodiment the ligand consists of 17 to 28 amino acids, preferably 20 to 24 amino acids, and more preferably 14 to 18 amino acids.

In one embodiment the present invention relates to a ligand as defined above comprising at least the amino acid sequence SEQ ID No. 3 or a biologically active derivative thereof, wherein the ligand selectively binds Aβ. In a preferred embodiment of the present invention, the ligand is selected from the group consisting of SEQ ID No. 2, SEQ ID No. 3, and biologically active derivatives thereof. In an even more preferred embodiment, the ligand has the amino acid sequence of SEQ ID No. 3 or a biologically active derivative thereof.

Further, in another embodiment the present invention relates to a ligand as defined above comprising at least the amino acid sequence SEQ ID No. 7 or a biologically active derivative thereof, wherein the ligand selectively binds HCC. In a preferred embodiment of the present invention, the ligand is selected from the group consisting of SEQ ID No. 6, SEQ ID No. 7, and biologically active derivatives thereof. In an even more preferred embodiment, the ligand has the amino acid sequence of SEQ ID No. 7 or is a biologically active derivative thereof.

As used herein the term "biologically active derivative" means any derivative of a molecule having substantially the same functional and/or biological properties of said molecule, such as binding properties, and/or the same structural basis, such as a peptidic backbone. Biologically active derivatives of the ligand according to the present invention also include functionally active derivatives which encompass any ligand with substantially the same biological function as the ligand according to the present invention. The amino acid sequences of the functionally active derivatives may contain deletions, additions and/or substitution of amino acids whose absence, presence and/or substitution, respectively, do not have any substantial negative impact on the activity of the ligand, e.g. amino acids which are located in a part of the amino acid sequence that does not contribute to the biological activity of the ligand. Minor deletions, additions and/or substitutions of amino acids of the respective ligand sequences which are not altering the biological activity of said ligand are also included in the present application as functionally active derivatives.

The present invention further relates to a ligand for selectively binding at least one non-complexed member of the β-amyloid polypeptide (Aβ)/human cystatin C (HCC)-complex consisting of the amino acid sequences SEQ ID No. 3 and/or SEQ ID No. 7, and optionally N- and/or C-terminal moieties having substantially no negative impact on said selective binding.

The ("artificial") ligands according to the present application can be produced using any suitable method known in the art. This may include any method known in the art for (i) the production of recombinant DNA by genetic engineering, e.g. via reverse transcription of RNA and/or amplification of DNA, (ii) introducing recombinant DNA into procaryotic or eucaryotic cells by transfection, e.g. via electroporation or microinjection, (iii) cultivating said transformed cells, e.g. in a continuous or batchwise manner, (iv) expressing the ligand, e.g. constitutively or upon induction, and (v) isolating said ligand, e.g. from the culture medium or by harvesting the transformed cells, in order to (vi) obtain a purified ligand, e.g. via anion exchange chromatography or affinity chromatography. Additionally, the ligands of the present invention can be produced by chemical synthesis.

Further, a ligand according to the present invention having molecular recognition structures and binding sites in the Aβ-HCC interaction complex can be determined using proteolytic excision and mass spectrometry techniques.

Techniques which may be employed for obtaining the ligand according to the present invention encompass electrospray (ESI) and/or MALDI mass spectrometry, both being "soft' ionisation techniques suitable to
(i) directly analyse intact protein-peptide complexes and
(ii) yield stable, intact and highly accurate molecular mass data of peptides and proteins.

In particular, the combination techniques which may be employed in the present invention comprise
(i) the formation of a selective immobilisation matrix of Aβ and HCC for studies of protein-peptide interactions,
(ii) specific affinity-binding of Aβ (1-40) to an HCC affinity-microcolumn and of HCC to an Aβ (1-40) affinity-microcolumn, followed by proteolytic digestion of the complex and the removal of unbound material by simple washing steps, and
(iii) release of the remaining, affinity-bound peptide fragments produced by the selective protease digestion at non-shielded regions using standard elution solvents, and their subsequent mass spectrometric structure identification.

Direct methods of chemical structure identification of biological ligand complexes such as protein-peptide interactions, receptor-ligand complexes and antigenic determinants (epitopes) in peptide/protein-antibody complexes using combination of selective proteolytic and affinity-mass spectrometric methods are known in the art, e.g. by combining isolation of antibody-bound peptides using immuno-affinity techniques followed by the precise identification of epitope peptides by mass spectrometry. Mass spectrometric methods can provide structural details on many levels, for different classes of biomolecules. Proteins now can be analyzed by mass spectrometry to reveal elemental composition, complete or partial amino acid sequences, post-translational modifications, protein-protein interaction sites and structures, and even provide insight in higher order structures. One key advantage of mass spectrometric analyses over other analytical techniques is its capability to study extremely small quantities of molecules with high sensitivity. The "soft-ionization/desorption" techniques such as electrospray (ESI) and matrix assisted laser desorption/ionization (MALDI) are major tools for these analysis capabilities. In the present invention, mass spectrometric methods can be used that provide a direct, molecular determination of the ligand according to the present invention.

The invention further relates to a method for detecting the absence or presence of at least one selected from a non-complexed member of the β-amyloid polypeptide (Aβ)/human cystatin C (HCC)-complex and Aβ/HCC-complex, comprising the steps of:
(a) incubating a system containing at least one of a non-complexed member of the Aβ/HCC-complex and Aβ/HCC-complex with at least one ligand as defined above under Aβ/HCC-complex member/ligand-complex forming conditions; and
(b) evaluating the Aβ/HCC-complex member/ligand-complex formed in step (a) qualitatively and/or quantitatively.

The method as defined above is preferably used *in vitro* or extracorporeal.

As used herein, the term "non-complexed member of the Aβ/HCC-complex" or "Aβ/HCC-complex member" includes Aβ, HCC, and biologically active derivatives thereof.

Accordingly, in a preferred embodiment the present invention relates to a method as defined above, wherein the non-complexed member of the Aβ/HCC-complex is Aβ, comprising the steps of
(a) incubating a system containing at least one Aβ with at least one ligand comprising at least the amino acid sequence SEQ ID No. 3 or a biologically active derivative thereof, preferably a ligand being selected from the group consisting of SEQ ID No. 2, SEQ ID No. 3, and biologically active derivatives thereof, more preferably SEQ ID No. 3 or a biologically active derivative thereof, under Aβ/ligand-complex forming conditions; and
(b) evaluating the Aβ/ligand-complex formed in step (a) qualitatively and/or quantitatively.

In another preferred embodiment the present invention relates to a method as defined above, wherein the non-complexed member of the Aβ/HCC-complex is HCC, comprising the steps of
(a) incubating a first system containing at least one HCC with at least one ligand comprising at least the amino acid sequence SEQ ID No. 7 or a biologically active derivative thereof, preferably a ligand being selected from the group consisting of SEQ ID No. 6, SEQ ID No. 7, and biologically active derivatives thereof, more preferably SEQ ID No. 7 or a biologically active derivative thereof, under HCC/ligand-complex forming conditions; and
(b) evaluating the HCC/ligand-complex formed in step (a) qualitatively and/or quantitatively.

The present invention further provides a method of diagnosing a neurodegenerative disease as defined herein comprising the step of detecting the absence or presence of at least one non-complexed member of the β-amyloid polypeptide (Aβ)/human cystatin C (HCC)-complex by
(a) incubating a first system containing at least one non-complexed member of the Aβ/HCC-complex, and a second system containing at least one ligand as defined above under Aβ/HCC-complex member/ligand-complex forming conditions; and
(b) evaluating the AβHCC-complex member/ligand-complex formed in step (a) qualitatively and/or quantitatively.

In one embodiment of the present invention, the system may be a naturally occurring system such as a solution selected from the group consisting of whole blood, serum, plasma, urine, bile, tissue, like e.g. bladder or kidney, and cerebrospinal fluid (CSF). Further, the system may comprise a solution derived from naturally occurring systems, e.g. a solution containing isolated blood compounds or processed blood products. In another embodiment of the present invention, the system may comprise cells expressing polypeptides, e.g. a cell culture system. Methods for obtaining the above systems are known in the prior art.

The Aβ/HCC-complex member/ligand-complex formed can be evaluated qualitatively and/or quantitatively by methods well known in the art. Examples for the evaluation of the above mentioned complex are, but not limited to the use of a labelled antibody directed against one or more ligands as defined above or non-complexed members of the Aβ/HCC-complex. Further, a labelled antibody directed against one or more ligands as defined above or non-complexed members of the Aβ/HCC-complex may be used or one or more ligands as defined above or non-complexed members of the Aβ/HCC-complex may be covalently linked to a detectable label which may also be any suitable detectable label known in the art. The detection method for measuring the detectable label can be, for example, selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of the person skilled in the art to choose the optimal parameters, such as buffer system, temperature and pH for the respective detection system to be used.

In one embodiment of the present application, the Aβ/HCC-complex can be detected using specific affinity-determination, like e.g. an ELISA, with corresponding ligand-specific antibodies.

In one embodiment of the present invention the ligands as defined above are immobilized on a solid substrate. In another preferred embodiment of the present invention the non-complexed members of the Aβ/HCC-complex are immobilized on a solid substrate. The term "substrate" does not have any specific limitations, and relates, for example, to an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextrane, agarose, chitin and polyamino acids, or an inorganic polymer, such as glass or metallohydroxide. The substrate can be in the form of a microcarrier, particles, membranes, strips, paper, film, pearls or plates, such as microtiter plates or microarrays. The term "microarray" as used herein means any arrangement of the antibodies or ligands in addressable locations on a substrate resulting in a so-called "biochip".

The ligands as defined above or the non-complexed members of the Aβ/HCC-complex can be immobilized on the substrate directly by covalent coupling or via a carrier such as a linker molecule or an antibody immobilized on the substrate. In one specific example of the present invention, the Aβ/HCC-complex member/ligand-complex formed may be detected using an Enzyme Linked Immunosorbent Assay (ELISA).

In another embodiment of the present invention the above system is a tissue sample, for example a tissue section, which may be fixed on a slide, and the Aβ/HCC-complex member/ligand-complex is detected using *in situ* hybridiziation techniques.

The present invention further relates to a use of the method as defined above for detecting the absence or presence of a pathological alteration of the central nervous system, preferably the brain. In a preferred embodiment the pathological alteration of the central nervous system is a plaque in the brain tissue.

Additionally, the present invention provides a pharmaceutical composition containing a therapeutically effective amount of a ligand as defined above and optionally a pharmaceutically acceptable carrier and/or diluent.

In another embodiment of the present invention, a ligand as defined above for use in the treatment of a neurodegenerative disease is provided. The terms "pharmaceutical composition" and "medicament" as used herein relate to any pharmaceutical composition comprising at least one ligand as defined above in a pharmaceutically effective amount. The pharmaceutical composition may further comprise a pharmaceutically acceptable carrier and/or diluent and/or salt and/or buffer and/or excepient.

The present invention further relates to the use of a ligand as defined above in the manufacture of a medicament for the treatment of a neurodegenerative disease in mammals as defined herein.

The present invention further relates to the use of the ligand as defined above as an inhibitor of neurodegenerative disorders.

According to one embodiment of the present invention, after administration to a patient the above identified ligands comprising at least the amino acid sequence SEQ ID No. 3 or a biologically active derivative thereof, preferably a ligand being selected from the group consisting of SEQ ID No. 2, SEQ ID No. 3, and biologically active derivatives thereof, more preferably SEQ ID No. 3 or a biologically active derivative thereof, attach to Aβ and inhibit Aβ-oligomerisation and fibril formation in said patient.

Further, according to another embodiment of the present invention, after administration to a patient the above identified ligands comprising at least the amino acid sequence SEQ ID No. 7 or a biologically active derivative thereof, preferably a ligand being selected from the group consisting of SEQ ID No. 6, SEQ ID No. 7, and biologically active derivatives thereof, more preferably SEQ ID No. 7 or a biologically active derivative thereof, attach to HCC and prevent the formation of an AβHCC-complex in said patient.

Additionally, the ligands according to the present invention provide new templates for designing efficient inhibitors of the Aβ-aggregation process in neurodegenerative diseases, particular Alzheimer's Disease (AD). In a preferred embodiment the neurodegenerative disease is AD.

According to the present invention, the medicament and the pharmaceutical composition may be administered by any administration route known in the art being suitable. The route of administration does not exhibit particular limitations and includes for example inhalation, systemic administration, e.g. by oral or intravenous route, and topic application, e.g. as an ointment. Optionally, suitable carriers may be used for administration. The medicament may be administered in any form known in the art, e.g. as a liquid, a powder, an aerosol, a capsule, or a tablet.

As used herein the term "neurodegenerative disorder" means any neurodegenerative disorder that can be treated or diagnosed using the ligand according to the present invention. In a preferred embodiment of the present invention the neurodegenerative disorder is selected from the group consisting of Parkinson-dependent neurodementias, AD, and AD-related diseases.

The present invention further provides the use of the ligand as defined above in the treatment of a patient having a neurodegenerative disorder as defined above.

Further, the term "treatment" as used herein relates to the prevention and/or eradication or amelioration of disease related symptoms and/or disease related disorders, like e.g. a reduced deposition of Aβ and/or HCC and/or the Aβ/HCC-complex and/or a reduced plaque formation. The reduction of the above symptoms and disorders as well as the success of a therapy of a neurodegenerative disorder as defined above, particularly AD or an AD-related disease in a patient by use of a ligand as defined above can be monitored using methods known in the art.

The treatment of a neurodegenerative disorder, particularly AD or an AD-related disease, as defined above can also be combined with any therapy known in the art for the therapy of a neurodegenerative disorder, particularly AD or an AD-related disease. Accordingly, the present invention also relates to the use of at least one ligand as defined above in a medicament which may also contain further active agents.

The term "patient" as used herein does not underly any specific limitation and includes mammals. In a preferred embodiment of the present invention, the patient is a human.

The present invention further relates to a method of treating a patient having a neurodegenerative disorder as defined above with at least one ligand as defined above. The present invention further relates to the use of the ligand as defined above in a method for diagnosing a neurodegenerative disorder as defined above.

The present invention further relates to the use of a ligand comprising at least the amino acid sequence SEQ ID No. 3 or a biologically active derivative thereof, preferably a ligand being selected from the group consisting of SEQ ID No. 2, SEQ ID No. 3, and biologically active derivatives thereof, more preferably SEQ ID No. 3 or a biologically active derivative thereof, for inhibiting Aβ-oligomerisation and/or fibril formation.

The present invention also relates to a pharmaceutical composition containing at least one ligand comprising at least the amino acid sequence SEQ ID No. 3 or a biologically active derivative thereof, preferably a ligand being selected from the group consisting of SEQ ID No. 2, SEQ ID No. 3, and biologically active derivatives thereof, more preferably SEQ ID No. 3 or a biologically active derivative thereof, for inhibiting Aβ-oligomerisation and/or fibril formation in a patient.

Additionally, the present invention relates to a method of treating a patient having a neurodegenerative disease being associated with Aβ-oligomerisation and/or fibril formation, comprising the step of adiministering a ligand comprising at least the amino acid sequence SEQ ID No. 3 or a biologically active derivative thereof, preferably a ligand being selected from the group consisting of SEQ ID No. 2, SEQ ID No. 3, and biologically active derivatives thereof, more preferably SEQ ID No. 3 or a biologically active derivative thereof.

The present invention also relates to the use of the ligand according to the present invention as an epitope for the production of antibodies binding specifically a non-complexed member of the Aβ/HCC-complex, preferably Aβ, HCC, and/or the AβHCC-complex. The present invention also relates to an antibody binding specifically a non-complexed member of the Aβ/HCC-complex, preferably Aβ, HCC, and/or the Aβ/HCC-complex, as well as to a process for obtaining an antibody binding specifically a non-complexed member of the Aβ/HCC-complex, preferably Aβ, HCC, and/or the Aβ/HCC-complex comprising the steps of (a) injecting a ligand as defined above into a suitable animal, for example a laboratory animal like e.g. a rabbit, a mouse, or a goat, and (b) isolating the antibody from the animal.

In a preferred embodiment the antibody is a therapeutic antibody for the immunotherapy of a neurodegenerative disorder as defined above. In a more preferred embodiment the antibody is an inhibitory agent which can be used for treating a neurodegenerative disorder as defined above.

In another embodiment the antibody is a diagnostic animal which can be used in the diagnosis of a neurodegenerative disorder as defined herein, preferably in the detection of the absence or presence of a non-complexed member of the Aβ/HCC-complex, preferably Aβ, HCC, and/or the Aβ/HCC-complex.

The present invention relates to a kit containing an antibody and/or a ligand as defined above for diagnosing a neurodegenerative disease in mammals. In a preferred embodiment of the present invention, the diagnosis comprises the step of detecting the presence or absence of at least one non-complexed member of the Aβ/HCC-complex, preferably Aβ, HCC, and/or the Aβ/HCC-complex to detect whether a patient has a risk of developing a neurodegenerative disorder, has a neurodegenerative disorder, or to monitor the course of a neurodegenerative disorder.

A kit as defined above may contain e.g. a buffer solution, a solid support as defined above, optionally having one or more ligands immobilized thereon, reaction containers, means for evaluating qualitatively and/or quantitatively a Aβ/HCC-complex member/ligand-complex or Aβ/HCC-complex member/antibody-complex formed, including buffers, enzymes, enzyme substrates, when appropriate.

The present invention provides means and methods which improve the detection of the presence of non-complexed member of the Aβ/HCC-complex by specifically targeting Aβ or HCC with the ligands as defined above having a high binding specificity. Accordingly, the present invention provides a highly reliable system for detecting the presence or absence of a non-complexed member of the Aβ/HCC-complex having a significantly increased sensitivity and, thus, enhancing the reliability of the detection process of said polypeptides. Accordingly, the present invention provides means for reducing the number of false negative results when testing clinical samples for the presence of non-complexed members of the Aβ/HCC-complex.

The present invention advantageously further provides means for the successful treatment of neurodegenerative disorders by providing binding non-complexed members of the Aβ/HCC-complex present in a body fluid of patient through the administration of the ligands as defined above.

In particular, it provides means for the direct identification, and access to molecular chemical structures, for the development of
(i) new therapeutic, neuroprotective ligand inhibitors of neurodegenerative disorders, particularly AD-related diseases;
(ii) new molecular diagnostics of neurodegenerative disorders, particularly AD and AD-related diseases;
(i) new therapeutic antibodies for immunotherapy of neurodegenerative disorders, particularly AD-related diseases.

Therefore, the present invention provides effective means for rapid, early diagnostics and molecular treatment of neurodegenerative diseases and provides new approaches of immunotherapy that may become highly efficient for the treatment of neurodegenerative disorders.

Additionally, the present invention provides a wide range of biochemical, analytical and biomedical applications of the ligands, methods, antibodies, and uses according to the present invention, including
(i) the use of the identified ligands for fundamental and mechanistic Aβ-aggregation studies, and the molecular understanding of amyloidogenic protein-peptide interaction processes;
(ii) the application of the ligands identified for affinity-isolation of specific proteins/peptides relevant for Aβ-binding from biological and cellular material as well as for corresponding proteomics studies;
(iii) the use of the binding sequences as templates for designing the new therapeutic/neuroprotective aggregation inhibitors.

The figures show:

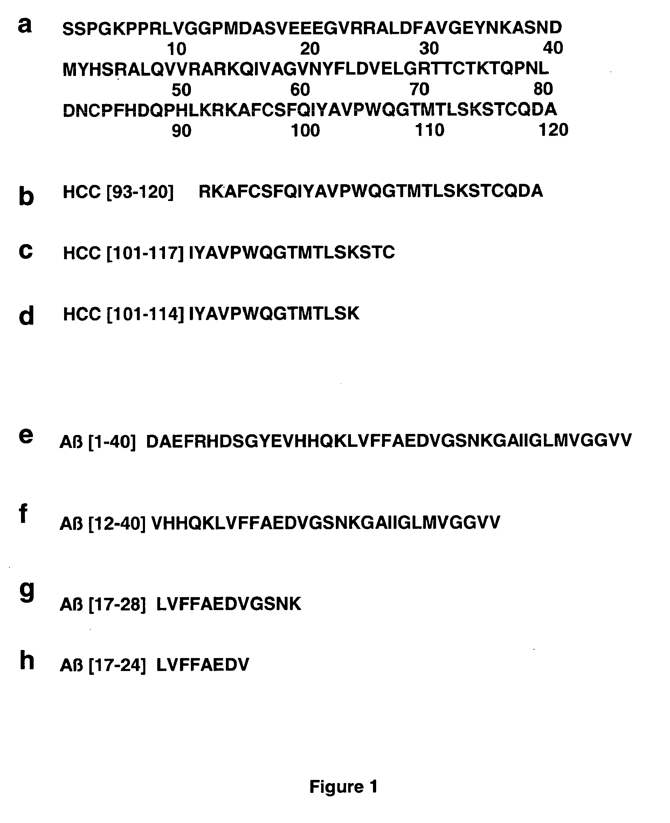

Figure 1 shows the amino acid sequence of
   (a) human cystatin C (HCC)
      SSPGKPPRLVGGPMDASVEEEGVRRALDFAVGEYNKASNDMYHSRALQVVR ARKQIVAGVNYFLDVELGRTTCTKTQPNLDNCPFHDQPHLKRKAFCSFQIYAV PWQGTMTLSKSTCQDA (SEQ ID No. 1),
   (b) HCC [93-120] RKAFCSFQIYAVPWQGTMTLSKSTCQDA (SEQ ID No. 2),
   (c) HCC [101-117] IYAVPWQGTMTLSKSTC (SEQ ID No. 3),
   (d) HCC [101-114] IYAVPWQGTMTLSK (SEQ ID No. 4),
   (e) β-amyloid (Aβ) Aβ [1-40]
      DAEFRHDSGYEVHHQKLVFFAEDVGSNKGAIIGLMVGGVV (SEQ ID No. 5),
   (f) Aβ [12-40] VHHQKLVFFAEDVGSNKGAIIGLMVGGVV (SEQ ID No. 6),
   (g) Aβ [17-28] LVFFAEDVGSNK (SEQ ID No. 7), and
   (h) Aβ [17-24] LVFFAEDV (SEQ ID No. 8).

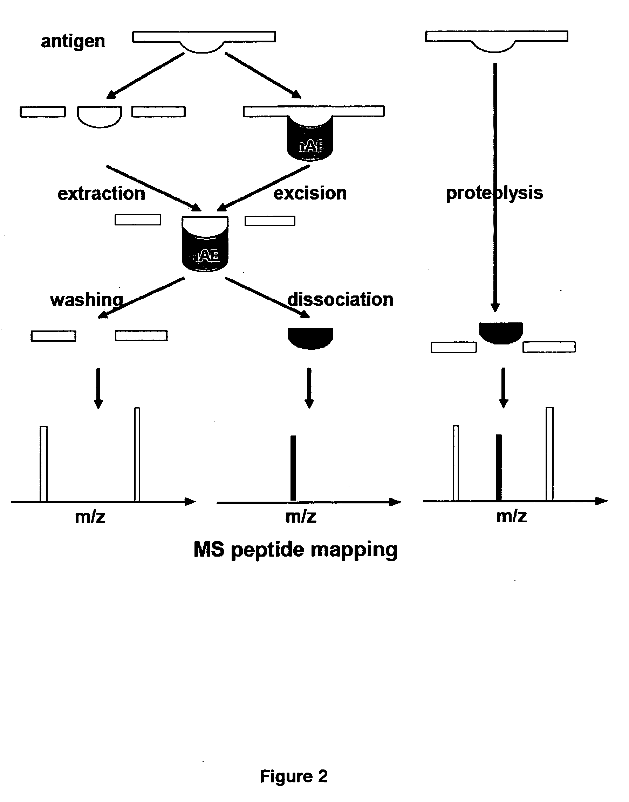

Figure 2 shows a general analytical scheme of epitope identification analysis using the combination of proteolytic excision/extraction and mass spectrometry (e.g. peptide mapping and sequence analysis). In epitope excision the antigen (ligand) is bound to the antibody (binder) and the immune complex is then proteolytically degraded by proteases. In the epitope extraction procedure, the antigen/ligand is first degraded and the proteolytic peptide mixture is then applied to the immobilized antibody/affinity.

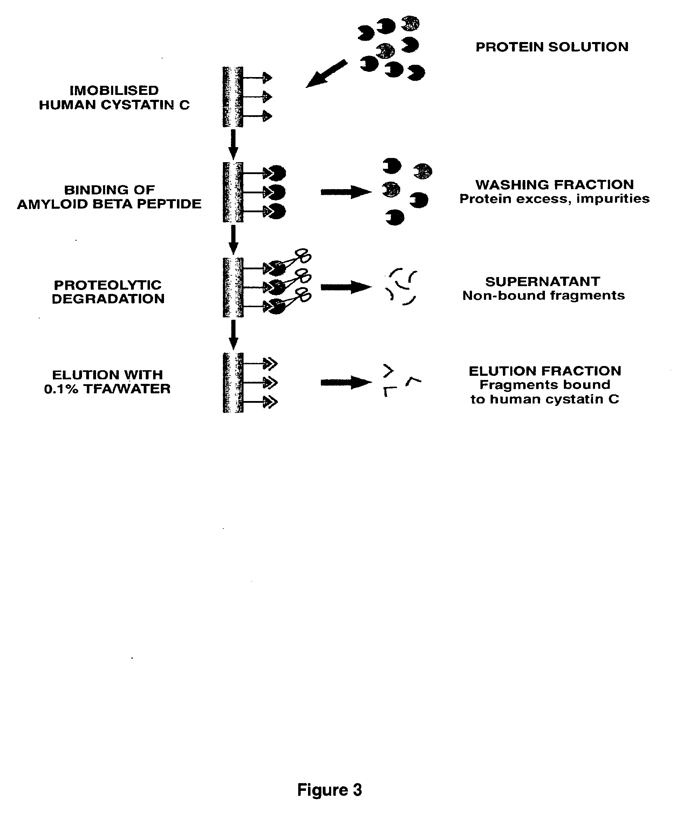

Figure 3 shows an analytical scheme of epitope excision-mas spectrometry employed in the determination of the HCC-Aβ interaction structures. In this protocol the Aβ-peptide was bound to the HCC-affinity column and then proteolytically degraded by different proteases. In the epitope extraction procedure, the Aβ-peptide was first degraded and the Aβ-peptide fragments were then applied to the column. Following specific affinity-elution, the resulting eluate fraction was analyzed by MS.

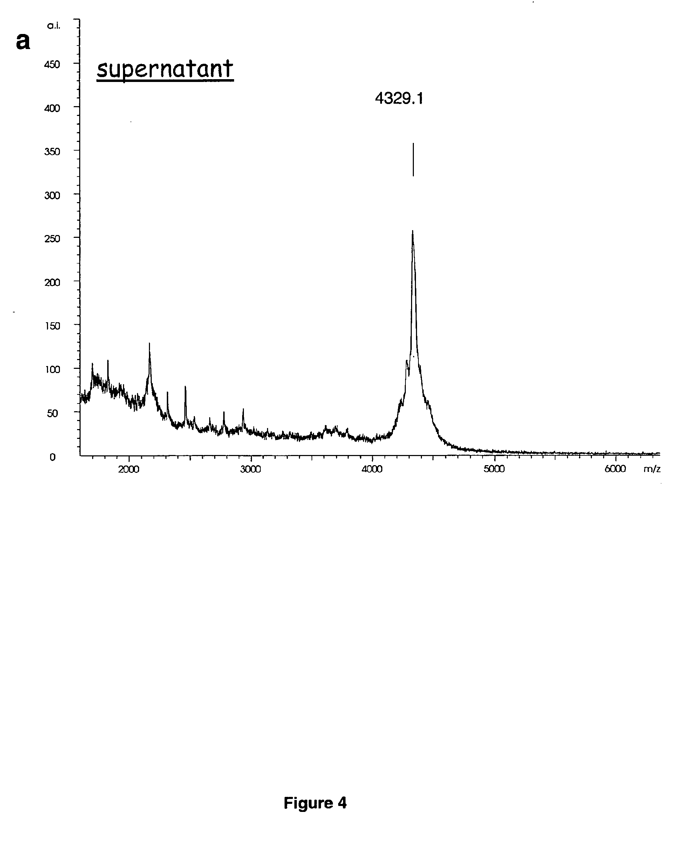

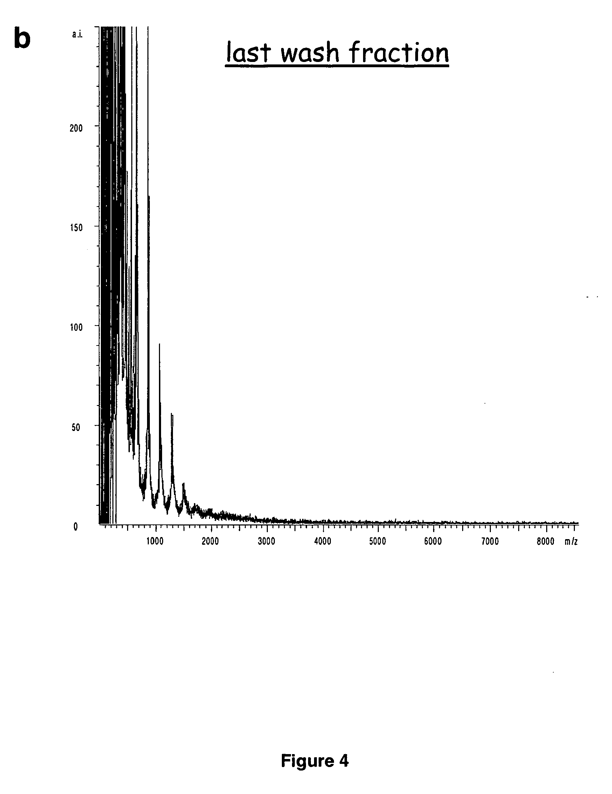

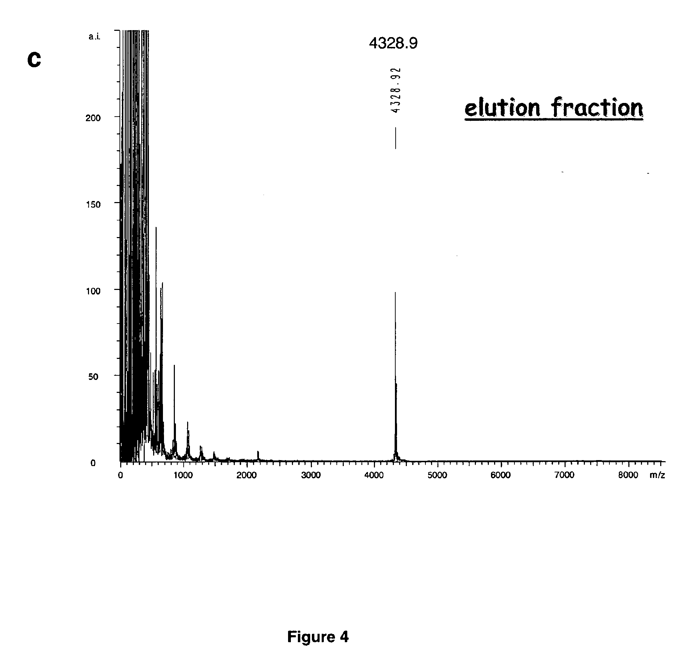

Figure 4 shows an affinity-MS of the Aβ-binding to HCC. 10 mg of Aβ (1-40) was added to the HCC-micro-column and incubated for two hours. After incubation, the supernatant fraction was collected, the column washed with 40 ml PBS and the last wash fraction collected for anaiysis. The elution was performed with 2 x 500 ml 0.1 % TFA (aqueous solution, pH 2).

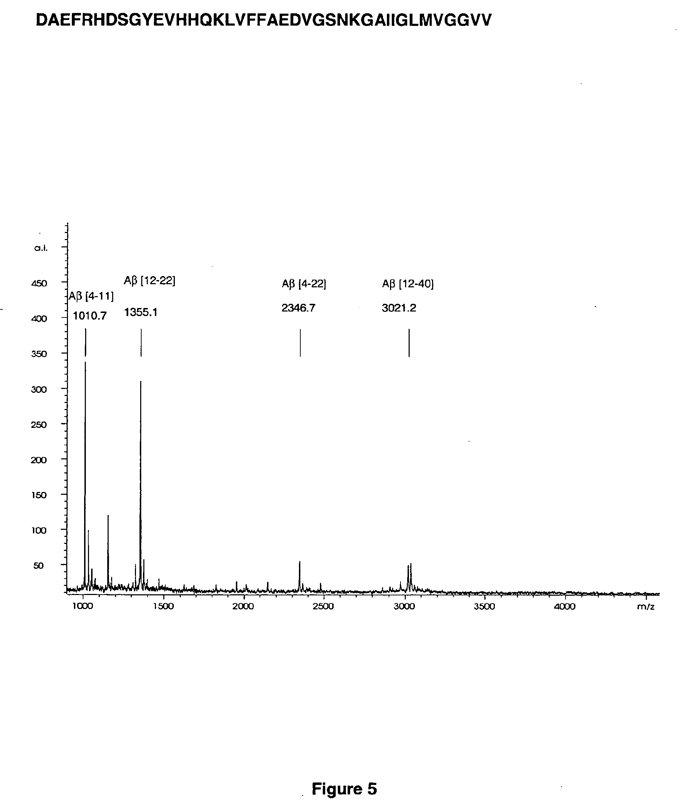

Figure 5 shows a MALDI-MS of the Glu-C protease digestion of Aβ (1-40) in solution. Digestion was performed with 10 µg Aβ (1-40) using Glu-C (enzyme:substrate ratio 1:50) for 2 hours at 37 °C in 50 µL PBS (50mM, pH 7.5).

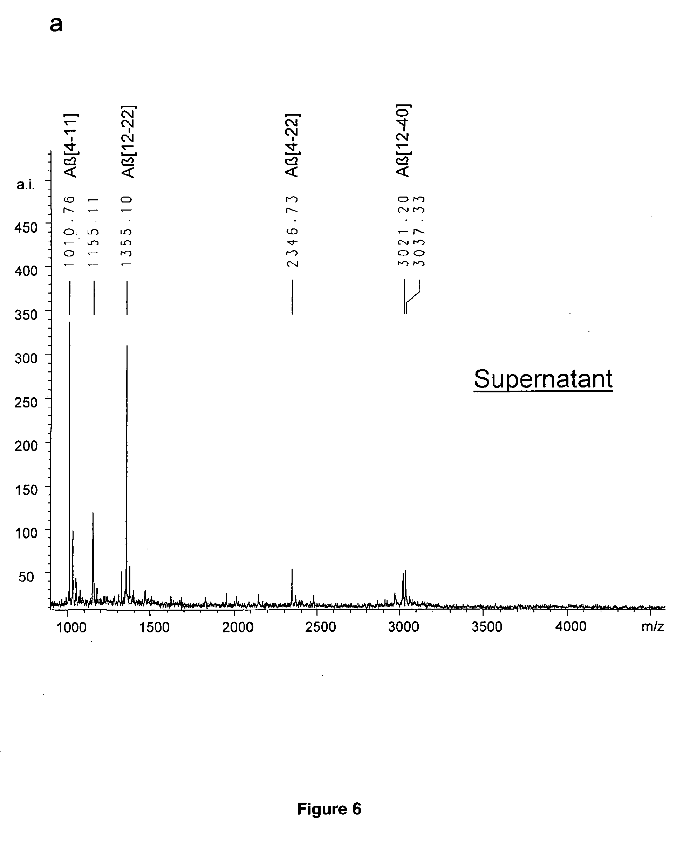

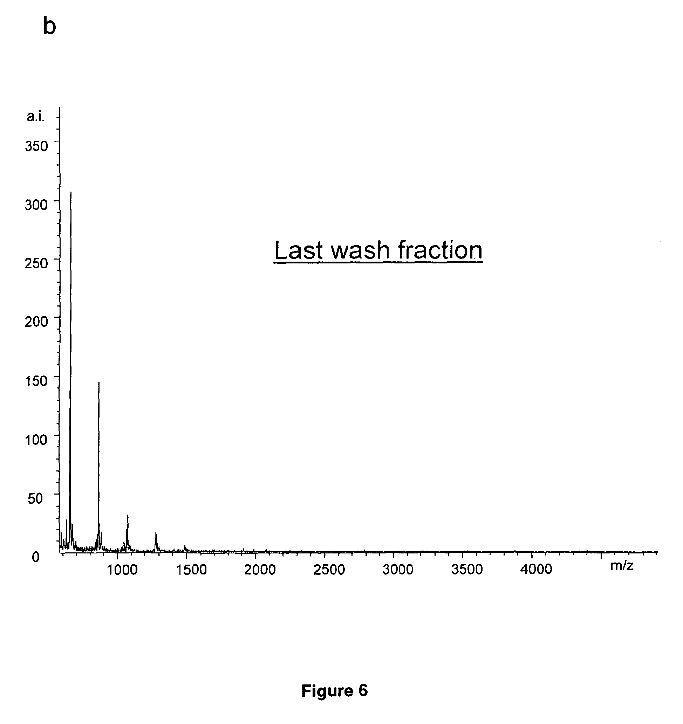

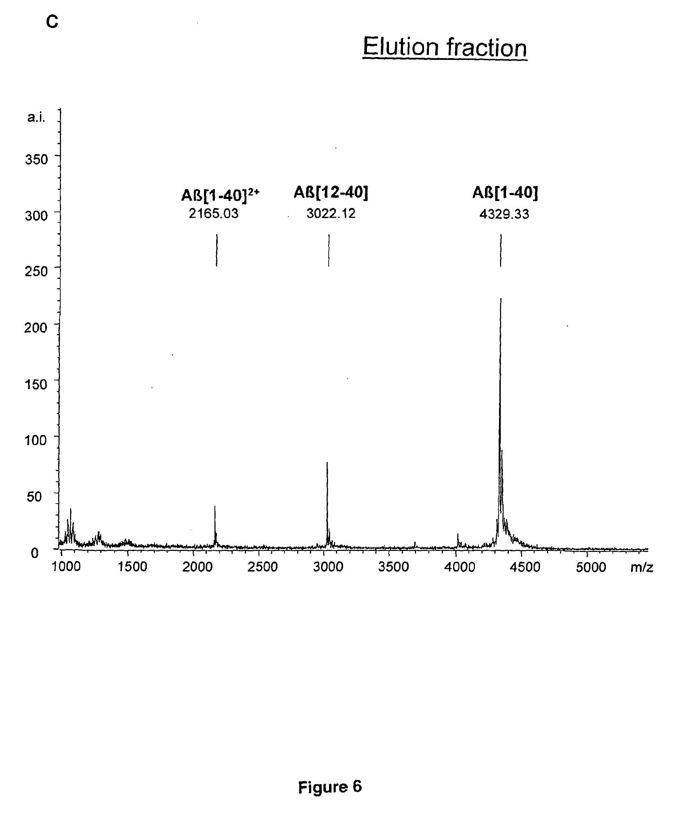

Figure 6 shows a determination of the Aβ-binding epitope to HCC by epitope excision-MALDI-MS with Glu-C protease. Approximately 10 mg Aβ (1-40) was bound to the HCC column by incubation for 2 hours in 50 mM PBS, pH 7.5. After washing off unbound material, the protease was added and the digestion was performed for 2 hours at 37°C. (a), Supernatant; (b), last washing fraction; (c), elution fraction.

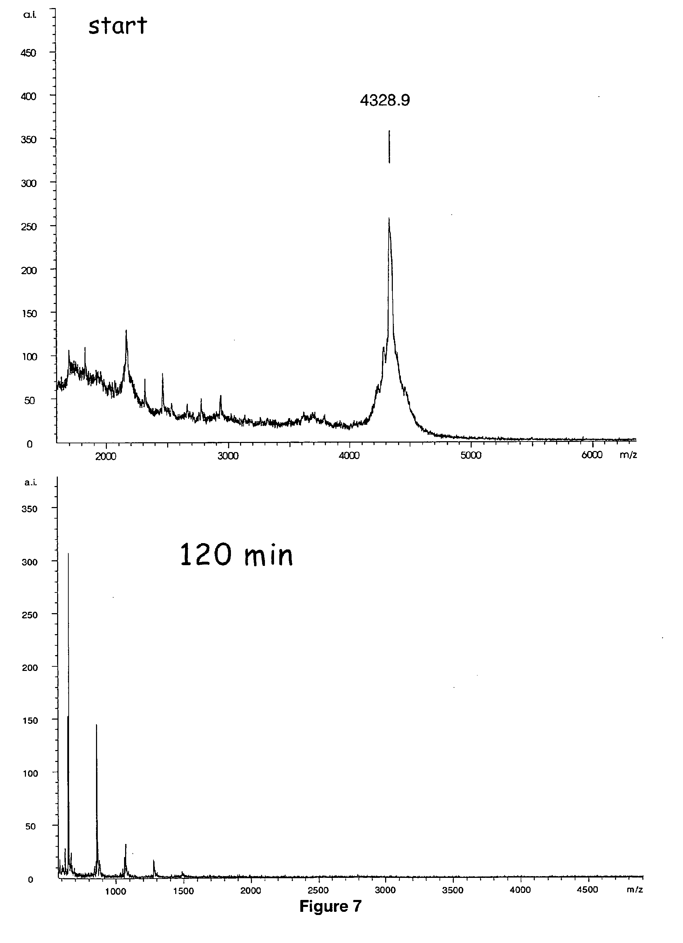

Figure 7 shows a MALDI-MS of the pronase digestion of Aβ (1-40) in solution. Digestion was performed with 10 µg Aβ (1-40) using pronase (enzyme:substrate ratio 1:50) for 2 hours at 40°C in 50 µL PBS (50mM, pH 7.5).

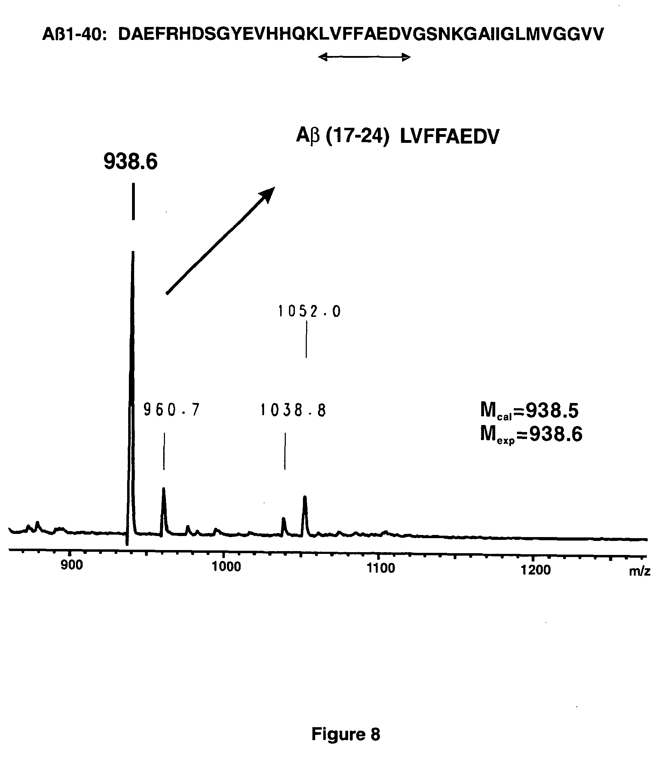

Figure 8 shows a MALDI-MS Analysis of elution fraction after epitope excision of Aβ (1-40) on immobilized HCC with pronase (see Figure 6 for details of experimental conditions).

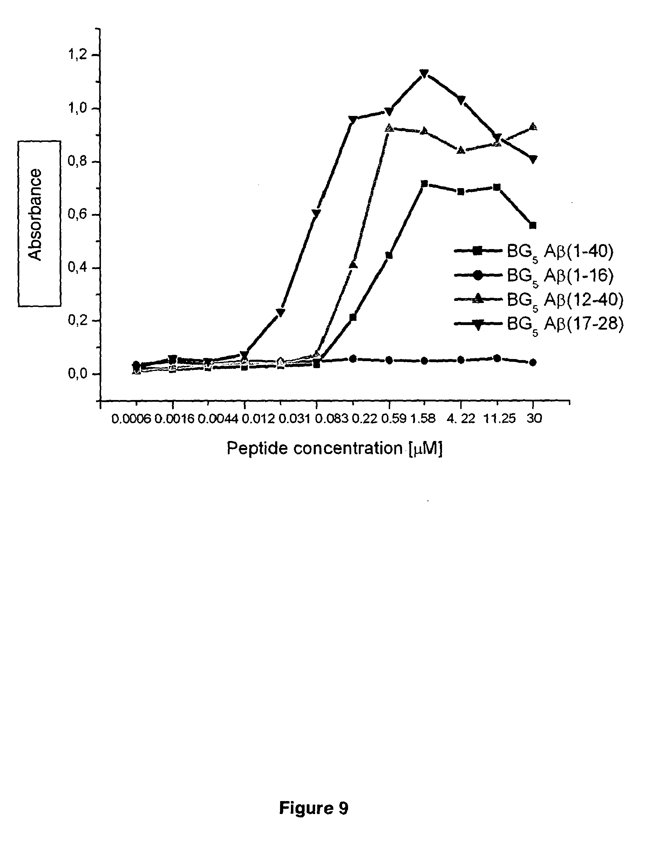

Figure 9 shows the binding of Aβ-peptides to HCC measured by ELISA. ELISA of biotinylated synthetic Aβ-peptides was carried out with Aβ (1-40) and the series of Aβ-peptides containing the epitope, as shown. The antibody used was anti-biotin HRP. ELISA was performed by applying 100 µl of human cystatin C (1 µM) in a 96-well microtiter plate. After blocking unspecific antigen binding sites using 5 % BSA in PBS-0.1 % Tween, the plate was coated with serial twofold dilutions of the biotinylated Aβ-peptides (Biotin-G₅ -Aβ). The HRP conjugated detection antibody was allowed to interact for 1 hr with the monoclonal antibody. Wells were washed three times with PBS and once with 50 mM sodium phosphate-citrate buffer, pH 5. 100 µl/well of 0.1 % o-phenylenediamine dihydrochloride (OPD) in phosphate-citrate buffer containing 0.006 % of H₂O₂ were added to the plates. The absorbance was measured at λ=450 nm on a Wallac 1420 Victor ELISA Plate Counter (Perkin Elmer).

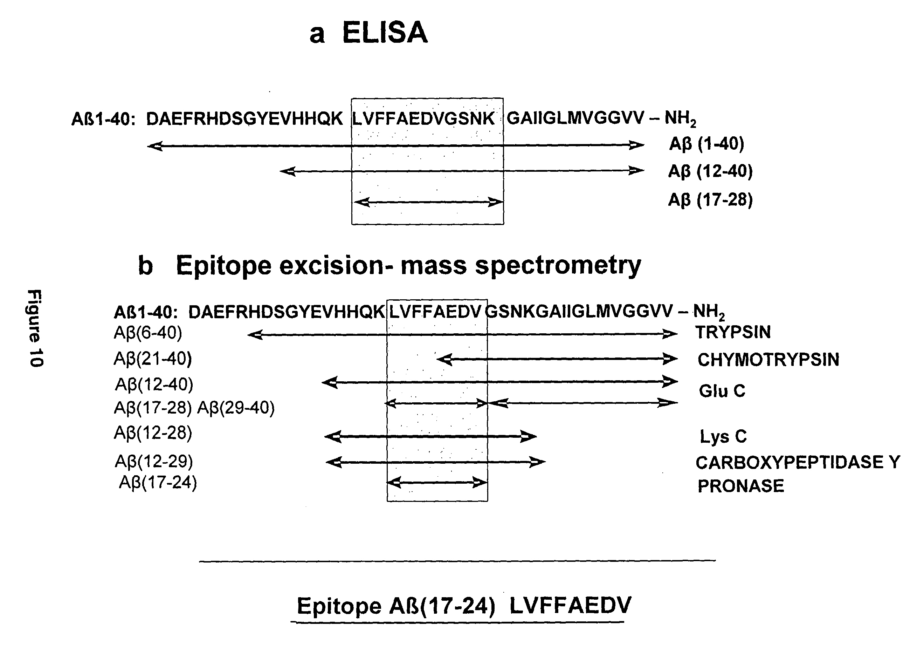

Figure 10 shows a summary and schematic representation of the results of affinity and epitope extraction/excision of Aβ (1-40). Affinity experiments showed the smallest binding Aβ-peptide to HCC as Aβ (17-28). Epitope Aβ extraction/excision-MS using trypsin, Glu-C, Lys-C, carboxypeptidase Y and pronase revealed the minimal epitope sequence, Aβ (17-24) LVFFAEDV.

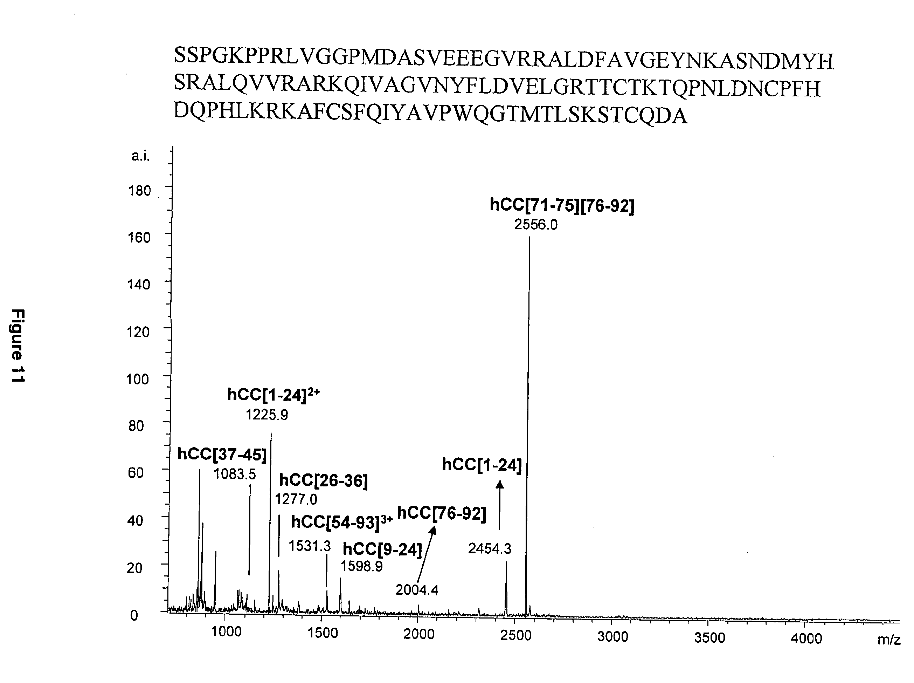

Figure 11 shows a MALDI-MS Analysis of the in solution-digestion of HCC with trypsin. 30 mg of HCC was dissolved in 50 ml of PBS, and the digestion performed for 2 hrs at 27 °C using an enzyme:substrate ratio of 1:20.

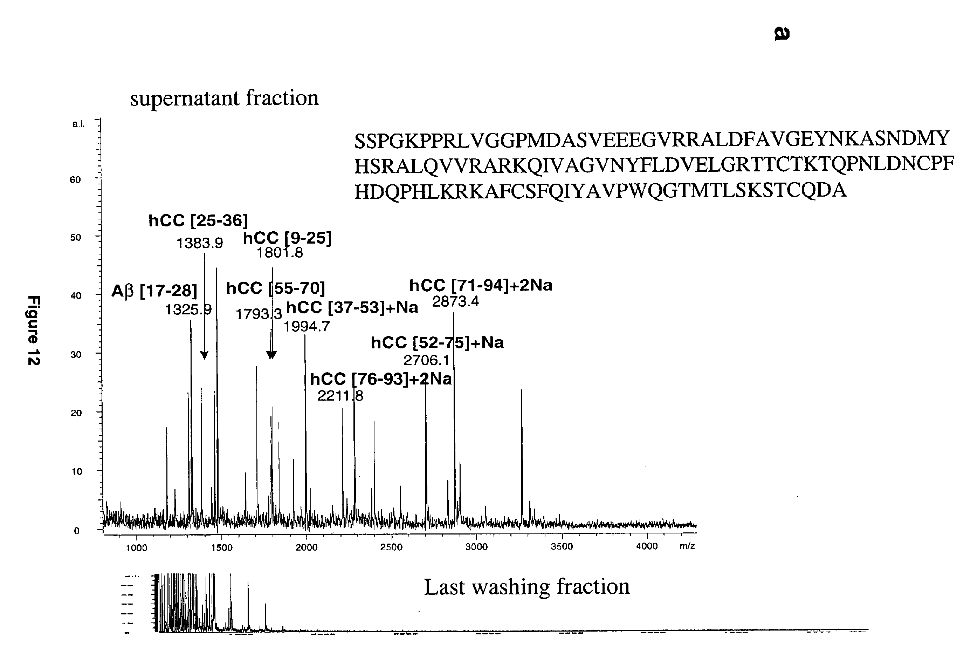

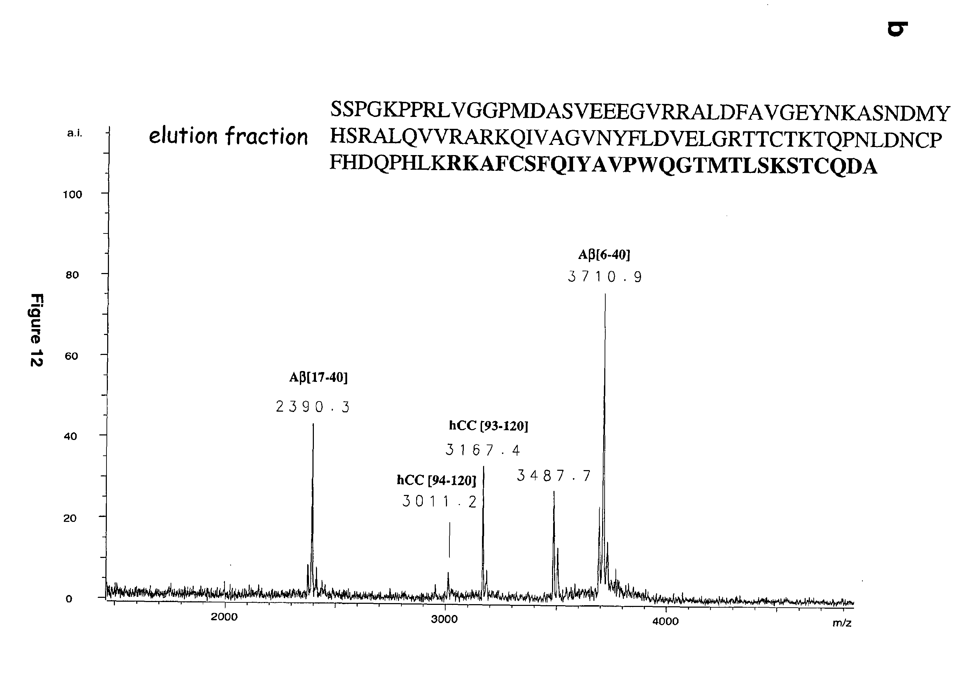

Figure 12 shows a mass spectrometric identification of the HCC binding epitope to Aβ (1-40) (a), supernatant fraction after trypsin digestion of HCC on immobilized Cys-Aβ (1-40). Lower trace, last washing fraction; (b), tryptic HCC fragments eluted after epitope excision from the Aβ (1-40)-HCC complex revealed two peptide fragments, HCC (93-120) and HCC (94-120), mw 3167.4 and 3011.2. Sequence of HCC, residues (87-120). Shaded sequence parts indicate the binding site, residues (101-117) (IYAVPWQGTMTLSKSTC). Proteolytic cleavages yielding fragments identified by epitope excision are indicated by solid arrows (trypsin, K91, R92, K93). Residues shielded from proteolytic digestion are indicated by broken arrows.

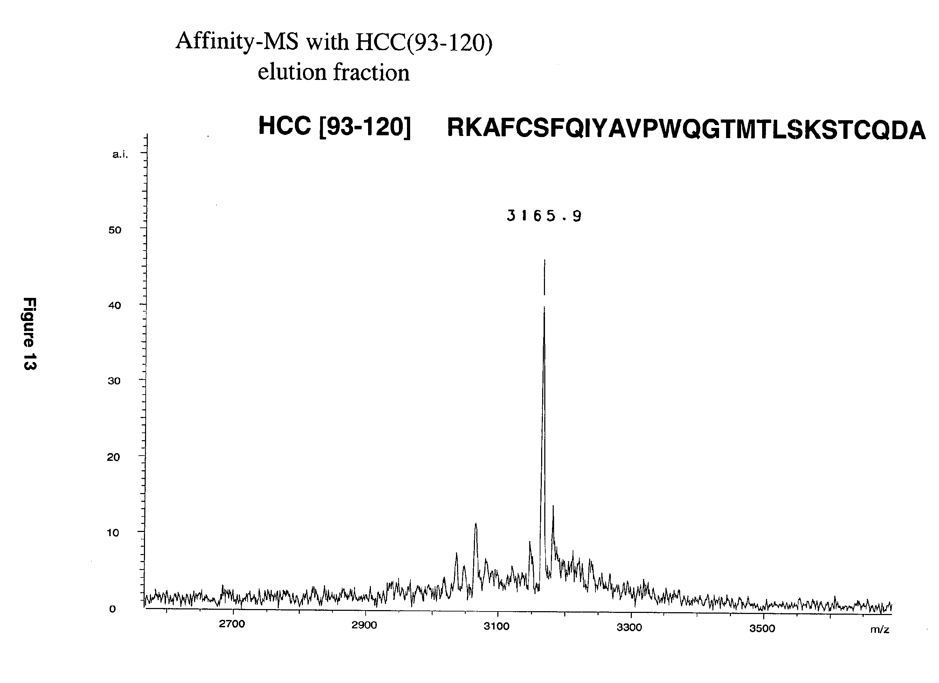

Figure 13 shows an affinity-MS analysis of the HCC (93-120)-peptide. 10 mg of HCC (93-120) was added to the Aβ-affinity column and incubated for two hours. After incubation, supernatant fraction was collected, the column washed with 40 ml PBS and the last wash fraction was collected. The elution was performed with 2 x 500 ml of 0.1 % TFA (aqueous solution, pH 2).

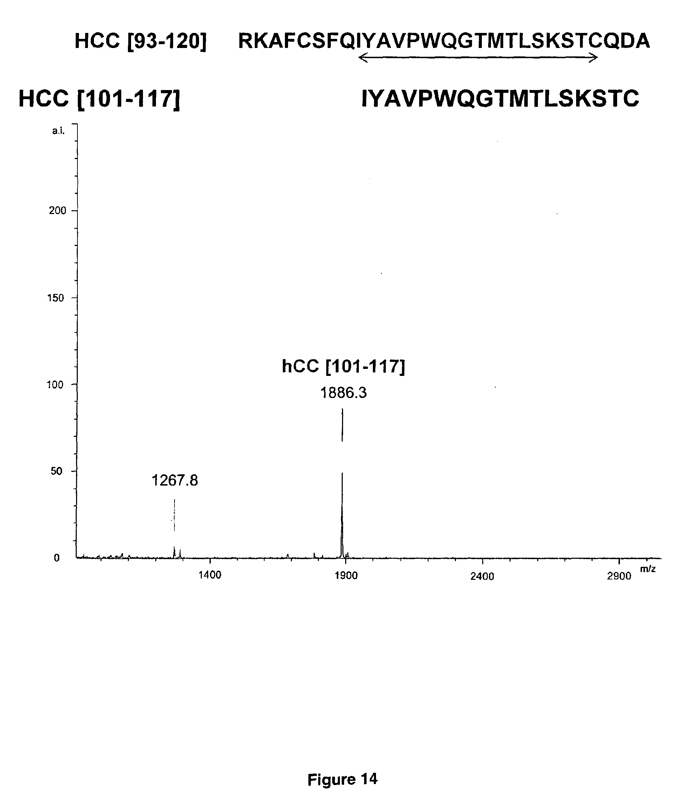

Figure 14 shows the identification of the HCC binding epitope fragment to Aβ (1-40) by epitope excision using pronase. The elution fraction upon pronase digestion provided a single peptide, HCC (101-117, mw 1886.3).

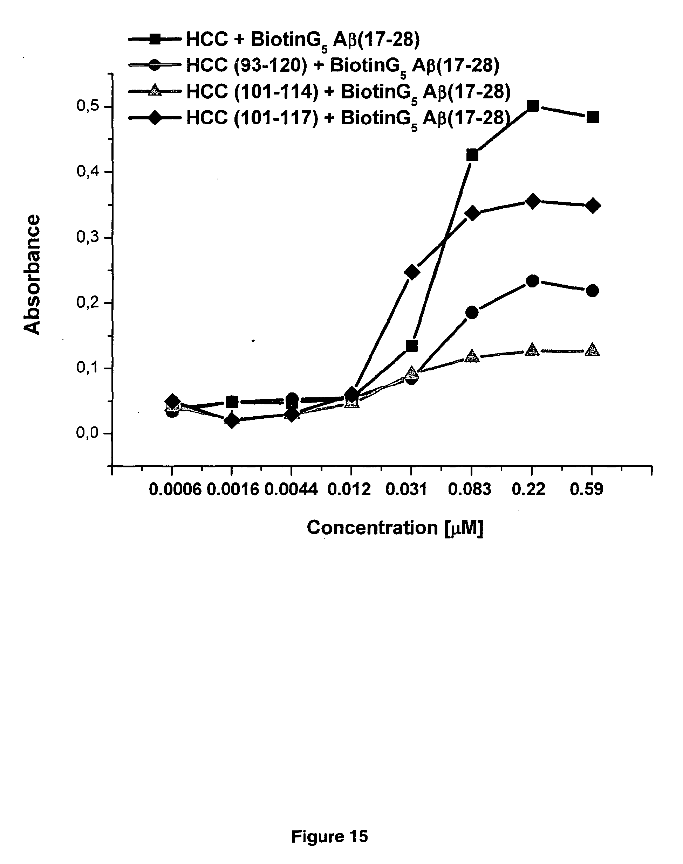

Figure 15 shows the binding of HCC-peptides to Aβ measured by ELISA. ELISA immunoassays were performed with 100 µl each of human cystatin C, HCC (93-120), HCC (101-114) and HCC (101-117) (1 µM) adsorbed onto a 96-well microtiter plates. After blocking unspecific binding sites using 5 % BSA in PBS-0.1 % Tween, the plate was coated with serial dilutions of the biotinylated Aβ-peptide (BiotinG₅ Aβ (17-28)) and incubated with HCC. The wells were washed eight times with PBS, and anti-biotin HRP was added to the plates as the detection antibody (dilution 1:5000). Wells were washed three times with PBS and once with 50 mM sodium phosphate-citrate buffer, pH 5, and 100 µl/well 0.1 % o-phenylenediamine dihydrochloride (OPD) in phosphate-citrate buffer containing 0.006 % of H₂O₂ was added to the plates. The absorbance was measured at 450 nm on a Wallac 1420 Victor ELISA Plate Counter (Perkin Elmer).

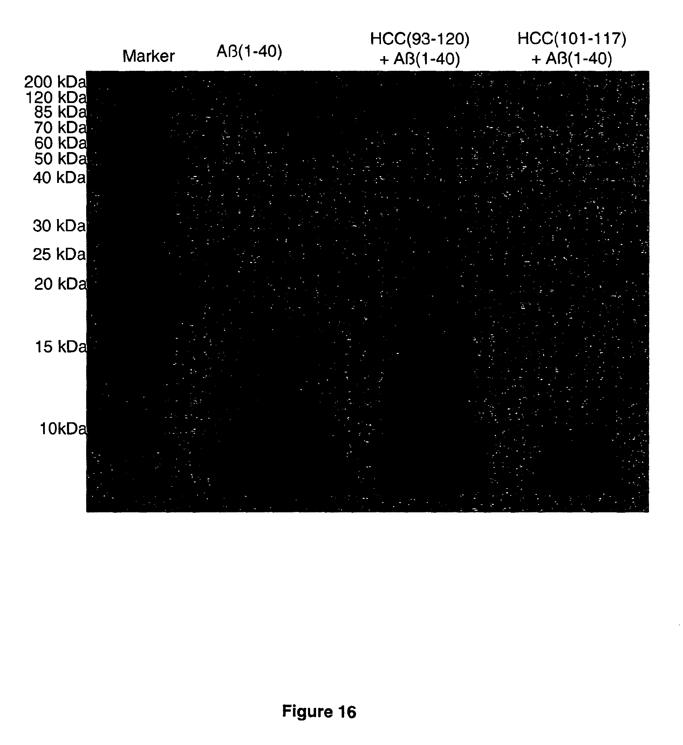

Figure 16 shows a photograph of a SDS-gelelectrophoresis showing the results of an inhibition assay of Aβ-oligomerisation by HCC-peptides. The gel was prepared using the mini-gel instrument MiniProtean from BioRad. The samples were dissolved in 50 µL of running buffer (25 % glycerol, 4 % SDS, Coomassie) and added to the 12 % gel (8.4 mL MilliO, 6 mL 4Xseparation buffer, 9.6 mL acrylamide solution, 125 µl APS, 20 µL TEMED). The power/PAC 1000 instrument from Bio-Rad at constant current was used for gel electrophoresis in two steps: (a) 60V when the samples were in the stacking gel; (b) 120V when the samples were in the separating gel.

The present invention will now be further illustrated in the following example without being limited thereto.

### Example

For the identification and determination of the protein-peptide complex binding sites and epitope structures, the epitope extraction/excison-mass spectrometry technique was generally employed. The general principle of this method is illustrated in Figure 2. For identification of the Aβ-and HCC epitopes, proteolytic excision-and extraction-MS techniques with the proteolytic enzymes trypsin, a-chymotrypsin, Lys-C-, Glu-C-, and Asp-N-protease, and pronase were employed in combination with MALDI-TOF-MS, and electrospray ionization-(ESI)-MS, and FTICR-MS using both MALDI and ESI ionisation. The high resolution FTICR-MS method typically yields mass determination accuracies at the low (1-2) ppm level, which enable the direct general identification of peptide components from the multi-component protein mixtures.

The experimental details of the epitope excision-mass spectrometric analysis with HCC are shown in Figure 3. In the first step, HCC was immobilized on NHS-activated sepharose 4B by dissolving approximately 100 mg of human cystatin C in 0.2 ml of 0.2 mol L⁻¹ NaHCO₃ and 0.5 mol L⁻¹ NaCl-coupling buffer (pH 8.3). The solution was added to dry NHS-activated Sepharose, and the coupling reaction performed for 1 hr at 25°C under vigorous shaking. The Sepharose-coupling product was loaded onto the micro-column and washed sequentially with blocking solution (0.1 mol L⁻¹ aminoethanol, 0.5 mol L⁻¹ NaCl, pH 8.3) and washing solution (0.2 mol L⁻¹ CH₃COONa 0.5 mol L⁻¹ NaCl, pH 4.0). The column was then incubated for 1 hr at room temperature with blocking solution followed by a second washing procedure, and the column finally washed again with 10 ml of 50 mmol L⁻¹ Na₂HPO₄, 150 mmol L⁻¹ NaCl, pH 7.5 and stored at 4°C. The activity of the column and binding affinity of Aβ (1-40) was confirmed by the affinity-MS experiment shown in Figure 4. The intact molecular ion of the Aβ-peptide was obtained exclusively in the affinity-elution fraction. The affinity-MS experiment was performed by applying 10 µg of Aβ-peptide onto the HCC microcolumn followed by incubation for 2 hrs at room temperature under gentle shaking. The supernatant fraction was then collected, the column washed with 40 ml PBS, unbound material removed and the last wash fraction collected for MS analysis. The Aβ-HCC complex was then dissociated by addition of 500 µl 0.1 % aqueous trifluoroacetic acid (pH 2) and the released peptide collected in an Eppendorf cup and lyophilized for mass spectrometric analysis (see Figure 4).

Epitope excision-MS experiments for identification of the Aβ-epitope binding to HCC (as shown in Figure 2) were performed by applying 10 µg of Aβ-peptides onto the HCC micro-column followed by incubation for 2 hrs at room temperature with gentle shaking. After removal of the unbound peptides, the HCC-Aβ complex was digested with trypsin at 27°C for 2 hrs and with Glu-C at 37°C for 2 hrs (enzyme to substrate ratio 1:50). The supernatant containing digested unbound peptide fragments was removed by washing with 40 ml PBS buffer, and the supernatant and last wash fractions was collected for the analysis. The complex was then dissociated by addition of 500 µl 0.1 % trifluoroacetic acid and the elution fraction collected in an Eppendorf cup and lyophilised for storage until the mass spectrometric analysis. Epitope extraction-MS experiments were performed in the same manner, with the proteolytic digest mixture applied to the antibody column and incubation for 2 hrs at room temperature. The MALDI-MS results obtained, in comparison to MALDI-MS of the digestion of Aβ (1-40) with Glu-C protease in solution are shown in Figures 5 and 6. The solution digestion of the Aβ-peptide showed cleavage at all expected residues, Glu-3, Glu-11 and Glu-21. In contrast, epitope-excision-MS provided only the N-terminal Aβ-fragments (Aβ (12-40)) while the Glu-21 residue was shielded from digestion, showing that this residue is part of the epitope bound to HCC. Corresponding results were obtained with trypsin as the protease.

The exact binding epitope to HCC was established by epitope excision-MS with pronase. Pronase is a non-specific protease that is essentially breaking down proteins into single amino acids, as shown for Aβ (1-40) which was completely digested in 2 hrs (Figure 7). Epitope excision-MS using pronase provided a single Aβ-peptide fragment as the minimal epitope eluted from the HCC column, which was identified as Aβ (17-24), LVFFAEDV (Figure 8). Corresponding proteolytic excision experiments with different proteases yielded identical results (trypsin, Glu-C, Lys-C, pronase) and confirmed that the N-terminal part of Aβ is not involved in the HCC binding (Figure 9). The mass spectrometric identification of the Aβ-epitope was in complete agreement with the ELISA binding affinity data obtained with synthetic Aβ-peptides, confirming that the epitope is located in the mid-C-terminal part (17-28) of Aβ (Figures 9, 10). Highest binding affinity was found for Aβ (17-28) as well as for the carboxy-terminal peptides Aβ (12-40) and the complete Aβ-sequence, while the N-terminal peptide Aβ (1-16) did not show any binding. In contrast to the affinity of Aβ (17-28), the synthetic Aβ (17-24)-peptide did not show affinity, indicating some conformational requirement for binding to HCC. For the Aβ-HCC complex, a specific ELISA mode was developed for determining the affinities of biotinylated Aβ to human cystatin C. In this assay, 100 µl of HCC (3 mg/ml) was adsorbed onto a 96-well microtiter plate. After blocking unspecific binding sites using 5 % BSA in PBS-0.1 % Tween, the plate was coated with serial dilutions of the biotinylated Aβ-peptides (Biotinyl-G₅-Aβ) and incubated with HCC. The wells were then washed extensively with PBS and anti-biotin HRP was added as detection antibody. No anti-HCC or anti-Aβ antibody was used in these experiments in order to avoid potential problems by possible overlapping of antibody-epitopes with the binding sites of the Aβ-HCC complex.

A similar methodology was employed for the identification of the binding "paratope" peptide on HCC by immobilization of the Aβ-peptide on an affinity microcolumn. Since previous studies in our laboratory had shown that NHS-activated sepharose is not suitable for immobilization of short peptides and possible peptide fragments might be formed in the case of immobilized Aβ (1-40) in epitope excision experiments, an UltraLink-lodoacetyl gel was used for immobilization to enhance the stability of the Aβ-affinity column. In this system the linker is attached to the peptide via a thioether bond. For immobilization, the Aβ (1-40)-peptide was prepared with an additional N-terminal Cys residue. Then 2 ml of an UltraLink lodoacetil Gel slurry (containing approximately 1 ml of gel) was placed in a column, left for 15 min and washed 5 times with 10 ml coupling buffer (50 mM Tris, 5 mM EDTA, pH 8.5). N-Cysteinyl-Aβ (1-40) (470 mg) was then added to the column, mixed at room temperature for 15 min and then incubated for 30 min. After draining the column was further washed 3 times with coupling buffer, blocked with L-Cys (50 mM L-Cys in coupling buffer; 15 min mixing and 30 min incubation without mixing), and finally the column was washed 3 times with 1 M NaCl, 5 times with storage buffer (100 mM PBS, 150 mM NaCl, pH 7.2) and stored at 4 °C.

For epitope-excision-MS using trypsin, tryptic digestion of HCC was first characterised in solution in order to characterize the proteolytic peptides (Figure 11). The MALDI-MS analysis of the tryptic HCC digest under standard conditions (30 µg protein in PBS, pH 7.5, enzyme to substrate ratio 1:50, 27 °C, 2 hours) provided almost the complete sequence coverage except for two N-and C-terminal fragments HCC (1-8) and (94-120). The epitope-excision-MS results, supernatant and affinity-elution fractions, are shown in Figure 12 a and b. While the supernatant fraction contained essentially all tryptic fragments observed by digestion in solution, the elution fraction showed only the two C-terminal fragments, HCC (93-120) and HCC (94-120), while the Lys-114 residue was not cleaved and thus shielded from digestion which showed that the Aβ-binding site is located at C-terminal sequence of HCC. This result was fully confirmed by synthesis and affinity-MS characterization of the HCC (93-120)-peptide which showed specific binding to the Aβ-affinity column (Figure 13).

The identification of the exact binding structure was obtained by epitope excision with pronase (see MALDI-MS of the elution fraction; Figure 14). Pronase digestion on the immobilized Cys-Aβ-peptide was performed with the complete HCC, and with the synthetic HCC (93-120)-peptide, which both provided identical results. In both cases the shortest peptide eluted from HCC-Aβ complex after pronase treatment was identified as HCC (101-117). Furthermore, affinity-MS and ELISA studies with the Aβ-affinity column and different HCC-peptides confirmed the fragment HCC (101-117) as an Aβ-binding sequence of human cystatin C. ELISA immunoassays were performed the human cystatin C, HCC (93-120), HCC (101-114) and HCC (101-117) (1 µM), as described in Figure 9 (see legends to Figures 9 and 15). These results (Figure 15) clearly show that the C-terminus in human cystatin is responsible for complex formation with Aβ, with the HCC (101-117) as the minimal binding peptide.

Inhibition studies of Aβ-oligomerisation and fibril formation were performed with intact HCC in comparison with the C-terminal HCC epitope as shown in Figure 16, using an *in vitro* assay of Aβ-oligomerisation. For formation of Aβ-oligomers, Aβ (1-40) was first dissolved in 100 % TFE to ensure re-formation of monomers, and the sample then lyophilized and redissolved in 100 % DMSO. The samples were then diluted to a final peptide concentration of 100 µmol/L by initial addition of ice-cold 50 mM phosphate buffer, pH 7.4, to a final DMSO concentration of 10 % (v/v). Different samples with the Aβ (1-40) only, and with HCC or HCC-peptides added (dissolved in 100 %TFE, lyophilized and redissolved in ice-cold 50mM phosphate buffer, pH 7.4) were incubated at 37°C for up to 76 hours. Prior to analysis by SDS-PAGE, samples were centrifuged at 13,000g for 5 min at room temperature and then lyophilized. These comparative analyses clearly show a time-and concentration-dependant, inhibitory effect of the HCC epitope peptides on the formation of Aβ-oligomers/aggregates, with highest relative effect obtained for the HCC (101-117)-peptide.

Based on the above presented results, e.g. two binding peptides of the specific interaction for the Aβ-HCC complex were identified:
(A) The Aβ-epitope Aβ (17-28) : ¹⁷LVFFAEDVGSNK²⁸
(B) The C-terminal HCC peptide: HCC (101-117)
   ¹⁰¹IYAVPWQGTMTLSKSTC¹¹⁷

## Claims

1. A ligand for selectively binding at least one non-complexed member of the β-amyloid polypeptide (Aβ)/human cystatin C (HCC)-complex, comprising at least one amino acid sequence selected from the group consisting of SEQ ID No. 2 and/or SEQ ID No. 3 and/or SEQ ID No. 6 and/or SEQ ID No. 7 and/or biologically active derivatives thereof.

2. The ligand according to claim 1, comprising at least the amino acid sequence SEQ ID No. 3 or a biologically active derivative thereof, wherein the ligand selectively binds Aβ.

3. The ligand according to claim 1, comprising at least the amino acid sequence SEQ ID No. 7 or a biologically active derivative thereof, wherein the ligand selectively binds to HCC.

4. A method for detecting the absence or presence of at least one selected from a non-complexed member of the β-amyloid polypeptide (Aβ)/human cystatin C (HCC)-complex and the Aβ/HCC-complex, comprising the steps of:
(a) incubating a system containing at least one of a non-complexed member of the Aβ/HCC-complex and the Aβ/HCC-complex with at least one ligand as defined in any of claims 1 to 3 under Aβ/HCC-complex member/ligand-complex forming conditions; and
(b) evaluating the Aβ/HCC-complex member/ligand-complex and/or Aβ/HCC-complex/ligand-complex formed in step (a) qualitatively and/or quantitatively.

5. The method according to claim 4, wherein the non-complexed member of the Aβ/HCC-complex is Aβ, and the system in step (a) contains Aβ and the ligand as defined in claim 2.

6. The method according to claim 4, wherein the non-complexed member of the Aβ/HCC-complex is HCC, and the system in step (a) contains HCC and the ligand as defined in claim 3.

7. A pharmaceutical composition containing a therapeutically effective amount of a ligand as defined in any of claims 1 to 3 and optionally a pharmaceutical acceptable carrier and/or diluent.

8. A ligand as defined in any of claims 1 to 3 for use in the treatment of a neurodegenerative disease in mammals.

9. The ligand according to claim 8, wherein the neurodegenerative disease is Alzheimer's Disease.

10. A kit containing a ligand as defined in any of claims 1 to 3.

11. The kit according to claim 10 for diagnosing a neurodegenerative disease in mammals.


SEQUENCE LISTING

<110>  Universität Konstanz

<120>  Neuroprotective Peptides

<130>  K 3470EU

<160>  8

<170>  PatentIn version 3.3

<210>  1
<211>  120
<212>  PRT
<213>  human

<400>  1

Ser Ser Pro Gly Lys Pro Pro Arg Leu Val Gly Gly Pro Met Asp Ala
1               5                   10                  15

Ser Val Glu Glu Glu Gly Val Arg Arg Ala Leu Asp Phe Ala Val Gly
            20                  25                  30

Glu Tyr Asn Lys Ala Ser Asn Asp Met Tyr His Ser Arg Ala Leu Gln
        35                  40                  45

Val Val Arg Ala Arg Lys Gln Ile Val Ala Gly Val Asn Tyr Phe Leu
    50                  55                  60

Asp Val Glu Leu Gly Arg Thr Thr Cys Thr Lys Thr Gln Pro Asn Leu
65                  70                  75                  80

Asp Asn Cys Pro Phe His Asp Gln Pro His Leu Lys Arg Lys Ala Phe
                85                  90                  95

Cys Ser Phe Gln Ile Tyr Ala Val Pro Trp Gln Gly Thr Met Thr Leu
            100                 105                 110

Ser Lys Ser Thr Cys Gln Asp Ala
        115                 120

<210>  2

<211>  28
<212>  PRT
<213>  human

<400>  2

Arg Lys Ala Phe Cys Ser Phe Gln Ile Tyr Ala Val Pro Trp Gln Gly
1               5                   10                  15

Thr Met Thr Leu Ser Lys Ser Thr Cys Gln Asp Ala
            20                  25

<210>  3
<211>  17
<212>  PRT
<213>  human

<400>  3

Ile Tyr Ala Val Pro Trp Gln Gly Thr Met Thr Leu Ser Lys Ser Thr
1               5                   10                  15

Cys

<210>  4
<211>  14
<212>  PRT
<213>  human

<400>  4

Ile Tyr Ala Val Pro Trp Gln Gly Thr Met Thr Leu Ser Lys
1               5                   10

<210>  5
<211>  40
<212>  PRT
<213>  human

<400>  5

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile

20                  25                  30

Gly Leu Met Val Gly Gly Val Val
        35                  40


<210>  6
<211>  29
<212>  PRT
<213>  human

<400>  6

Val His His Gln Lys Leu Val Phe Phe Ala Glu Asp Val Gly Ser Asn
1               5                   10                  15


Lys Gly Ala Ile Ile Gly Leu Met Val Gly Gly Val Val
            20                  25


<210>  7
<211>  12
<212>  PRT
<213>  human

<400>  7

Leu Val Phe Phe Ala Glu Asp Val Gly Ser Asn Lys
1               5                   10


<210>  8
<211>  8
<212>  PRT
<213>  human

<400>  8

Leu Val Phe Phe Ala Glu Asp Val
1               5